Europäisches Patentamt

European Patent Office

Office européen des brevets

(19)

(11) Veröffentlichungsnummer: **0 207 356**
**A2**

(12) **EUROPÄISCHE PATENTANMELDUNG**

(21) Anmeldenummer: 86108230.3

(22) Anmeldetag: 16.06.86

(51) Int. Cl.⁴: **C 12 N 5/00**

(30) Priorität: 28.06.85 DE 3523202

(43) Veröffentlichungstag der Anmeldung:
07.01.87 Patentblatt 87/2

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Hübner, Günter E., Dr.
Ursulastrasse 7
D-5600 Wuppertal 1(DE)

(54) Verfahren zur Anzucht und Expansion empfindlicher Hybridome.

(57) Die Erfindung betrifft ein Verfahren zur Anzucht und/oder Expansion von Hybridomzellen, insbesondere von empfindlichen wie z.B. Hybridomzellen humanen Ursprungs, und die Verwendung von konditionierten Zellkulturmedien für dieses Verfahren.

Das konditionierte Medium ist ein zellfreies Medium und wird mit Hilfe von geeigneten Zellen gewonnen wie z.B. Milzzellen oder Lymphknotenzellen oder Thymozyten von Mäusen.

EP 0 207 356 A2

BAYER AKTIENGESELLSCHAFT    5090 Leverkusen, Bayerwerk
Konzernverwaltung RP
Patentabteilung            Bu/Kü-c


Verfahren zur Anzucht und Expansion empfindlicher
Hybridome

Die Erfindung betrifft ein Verfahren zur Anzucht und/oder
Expansion von Hybridomzellen, insbesondere von empfindlichen wie z.B. Hybridomzellen humanen Ursprungs, und die
Verwendung von konditionierten Zellkulturmedien für dieses
Verfahren.

Das konditionierte Medium ist ein zellfreies Medium und
wird mit Hilfe von geeigneten Zellen gewonnen wie z.B.
Milzzellen oder Lymphknotenzellen oder Thymozyten von
Mäusen.

Durch die Fusion von Zellen kann eine Neukombination von
Eigenschaften erreicht werden. So wurden in den letzten
Jahren häufig z.B. B-Lymphozyten mit lymphozytären Tumorzellen zu B-Zell-Hybridomen fusioniert. Dies führte zur
Entwicklung monoklonaler Antikörper, die in der Medizin
und in der biologischen Grundlagenforschung viele neue
Möglichkeiten eröffnen.

Le A 23 918 - Auslandstext

Als besonders schwierig hat es sich bisher erwiesen, nach der Herstellung humane Hybridome in großer Zahl und in kurzem Zeitraum routinemäßig aufzuziehen. Diese besonders empfindlichen Zellen lassen sich durch das erfindungsgemäße Verfahren in einer mit Hybridomen anderer Spezies (z.B. Maus) vergleichbar großen Zahl und kurzen Zeit aufziehen. Dazu werden den Kulturen konditionierte Medien zugegeben, die Gemische von Faktoren enthalten (sog. Lymphokine), die auf lymphozytäre Zellen einen stimulierenden Einfluß ausüben können.

Häufig sind Hybridome aufgrund der erfolgten Neukombination des genetischen Materials und den daraus folgenden zellulären Regulationsereignissen sehr empfindlich in bezug auf ein Überleben und Anwachsen in Kultur. Dies trifft insbesondere auch für humane Hybridome zu, die in einer Größenordnung von bestenfalls $10^{-5}$ bis $10^{-6}$ auf die zur Fusion eingesetzten Zellen anwachsen (R. J. Cote et al., Fed. Proc. 43, 2465-2469, 1984), während dies bei Maus-Hybridomen in der Regel ein Wert von ca. $10^{-4}$ bis $10^{-5}$ ist (W. Gerhard et al., PNAS 75, 1510-1514, 1978).

Zur Stimulation für die Aufzucht von Hybridom-Klonen wurden verschiedentlich humane Nabelschnur-Endothelzellen oder Kulturüberstand dieser Zellen zu den Kulturen zugegeben, wodurch eine Verdoppelung der Zahl an Klonen erreicht werden konnte (G.C.B. Astaldi et al., J. Immunol. 125. 1411-1414, 1980).

Le A 23 918

Zur Selektion von Hybridomzellen bei der Anzucht wird ein geeignetes Selektionsmedium verwendet, in dem die enzymdefekten, nicht fusionierten Tumorzellen und die Lymphozyten absterben. Als Selektionsmedien werden gewöhnlich entweder das sogenannte HAT-Medium mit den Zusätzen Hypoxanthin (H; $10^{-4}$ M), Aminopterin (A: $4\times10^{-7}$ M) und Thymidin (T; $1,6 \times 10^{-5}$ M) nach Littlefield eingesetzt (J.W. Littlefield, Science 145, 708-710, 1964) oder das sogenannte HAZA-Medium mit den Zusätzen Hypoxanthin (H; 50 µM) und Azaserin (Aza; 10 µm) nach Buttin et al. (G. Buttin et al., In: F. Melchers, M. Potter & N. Warner, Lymphocyte Hybridomas, Springer Verlag 1979, 27-36). Als Basis-Kulturmedium für diese Zusätze können verschiedene Medien wie z.B. RPMI 1640 oder DMEM/F12 mit 5-10 % fötalem Kälberserum (FCS) verwendet werden.

Überraschenderweise wurde nun festgestellt, daß durch die Verwendung von zellfreien konditionierten Zellkulturmedien bei der Anzucht und/oder Expansion von Hybridomzellen insbesondere humanen Ursprungs sich die Zahl der anwachsenden Klone deutlich erhöht, d.h. die konditionierten Medien besitzen einen stimulierenden Effekt auf die Proliferation und damit auf das Anwachsverhalten von Hybridomzellen. Das ist um so überraschender, wenn man berücksichtigt, daß für die Fusion Tumorzellen (z.B. Myelomzellen) eingesetzt werden, die der zellulären Wachstumsregulation nicht mehr gehorchen. Außerdem geht aus der Literatur hervor, daß konditionierte Medien keinen Einfluß auf das Anwachsen von humanen Hybridomzellen besitzen (R.J. Cote, et al, Fed. Proc. 43, 2465-2469 , 1984).

Le A 23 918

Die Erfindung betrifft also ein Verfahren zur Förderung der Proliferation bei der Anzucht und/oder Expansion von Hybridomzellen, wobei man die mit bekannten Verfahren fusionierten Zellen mit einem Zellkulturmedium aufnimmt, in geeignete Zellkulturgefäße aussät und unter geeigneten Bedingungen bebrütet und dem Zellkulturmedium ein mit geeigneten Zellen konditioniertes Zellkulturmedium zugesetzt wird.

Durch das erfindungsgemäße Verfahren wird die Zahl der anwachsenden Klone und die Geschwindigkeit des Klonwachstums wesentlich erhöht. Hergestellt werden die Hybridome durch Fusion von Tumorzellen (z.B. Myelomzellen) mit Lymphozyten.

Die Fusion kann mit literaturbekannten Verfahren (z.B. mit Hilfe von Polyethylenglykol oder Elektrofusion) durchgeführt werden. Sofort nach der Fusion werden die Zellen mit konditioniertem Medium in Kultur gebracht und inkubiert.

Konditionierte Medien können verschiedenen Ursprungs sein. So eignen sich beispielsweise durch Thymozyten von jungen Mäusen (höchstens 2 Wochen alt) konditionierte Medien (R.A. Luben & M.A. Mohler, Mol. Immunol. 17, 635-639, 1980) genauso wie Medien, die durch Zellen aus Lymphknoten oder Milz autoimmunkranker Mäuse (z.B. homozygote LPR-Mäuse) konditioniert wurden und B-Zell-Differenzierungsfaktoren enthalten (G.J. Prud'Homme et al., J. Exp. Med. 157, 730-742, 1983) oder solche Medien, die durch Maus-Milzzellen konditioniert wurden, nachdem diese Zellen

Le A 23 918

zuvor mit einem Mitogen wie z.B. Concanavalin A stimuliert worden waren (E. Pure et al., J. Immunol. 127, 1953-1958, 1981). Diese konditionierten Medien bewirken, daß die Proliferation der Hybridome innerhalb einer Woche einsetzt, die Klone somit bereits nach 10-14 Tagen gezählt werden können, und daß die Klonfrequenz bis zu $10^{-4}$ bis $10^{-5}$ inokulierter Zellen beträgt.

Der Anteil des konditionierten Kulturmediums am gesamten Medium hängt im wesentlichen von den Bedürfnissen der Hybridomzellen ab. Er liegt üblicherweise zwischen 10 % und 100 %. Ein Anteil von ca. 50 % wird bevorzugt.

Zusätzlich zur Stimulation der initialen Proliferation der Hybridome können die Lymphokin-haltigen Medien auch noch äußerst erfolgreich bei der Expansion von Klonen eingesetzt werden: Die anfängliche Kultivierung von Hybridomen erfolgt in der Regel in einem geringen Kulturvolumen. Beim Übertragen der Klone z.B. von Mikrotiternäpfen (bis 0,2 ml Volumen) in 1 ml-Kulturgefäße und anschließend in 10 ml-Kulturflaschen wird das jeweils verwendete konditionierte Medium in absteigender Konzentration zugesetzt, z.B. 50 %, dann 20 %, anschließend 5 %: Erst in noch größerem Volumen als 10 ml kann auf die Zusätze verzichtet werden. Auf diese Weise können bei empfindlichen Hybridomen gegebenenfalls bis zu 80 % aller Klone expandiert werden, während ohne Zusatz Lymphokin-haltiger Medien häufig nur etwa 10 % aller Klone expandiert werden können. Der Vorteil einer solchen Expansionsprozedur liegt darin, daß die Hybridomzellen allmählich an ein Kulturmedium ohne Lymphokine adaptiert werden und nicht nach dem Umsetzen in ein

Le A 23 918

anderes Gefäß und der damit verbundenen mechanischen Beanspruchung noch zusätzlich durch ein Medium ohne Lymphokin-Zusätze so in ihrer zellulären Integrität beeinträchtigt werden, daß sie dies nicht überleben.

Ein großer Vorteil des erfindungsgemäßen Verfahrens liegt darin, daß die Aufzucht empfindlicher Hybridome durch Zugabe von Stoffen wesentlich verbessert wird, die nicht von der gleichen Spezies wie die Hybridome stammen müssen (z.B. Lymphokine aus Mäusen für humane Hybridome). Sodann kann mit verschiedenen Lymphokin-haltigen Medien gearbeitet werden, deren Präparation in jedem Zellkulturlabor routinemäßig leicht durchführbar ist.

Le A 23 918

Beispiel

Jeweils $10^7$ WI-L2cl3-Zellen (humane Myelomzellen mit Enzymdefekt) in spät-logarithmischer Phase und unstimulierte oder mit Pokeweed Mitogen stimulierte humane mononukleäre Zellen aus dem peripheren Blut (nach Ficoll-Trennung) wurden gemischt und anschließend zentrifugiert. Dem Zellpellet wurden 2 Pulse mit 5 KV/cm appliziert (Dauer 30 µsec: Zeitintervall 1 Min.) und dann 1 ml Kulturmedium (RPMI 1640 oder DMEM/F12 1:1 mit 10 % fötalem Kälberserum (FCS)) zugegeben. Nach 1h erfolgte die Aussaat der Zellen in Mikrotiterplatten (Kulturvolumen 0,2 ml pro Napf). Die Zusammensetzung des Aufzucht-Mediums war: RPMI 1640 oder DMEM/F12 1:1 supplementiert mit Penicillin (100 U/ml), Streptomycin (100 µg/ml), L-Glutamin (2 mM), Hepes-Puffer (4 mM), 2-Mercaptoethanol (50 µM) und 7,5-10 % FCS; zusätzlich Hypoxanthin (50 µM) und Azaserin (10 µM) (G. Buttin et al. In: F. Melchers, M. Potier & N. Warner, Lymphocyte Hybridomas, Springer Verlag 1979, 27-36). Verschiedene konditionierte Medien wurden zusätzlich in 50 %iger Konzentration zugegeben (Thymozyten-konditioniertes Medium (TCM: R.A. Luben & M.A. Mohler, Mol. Immunol. 17, 635-639, 1980); Lymphknoten-konditioniertes Medium (LNCM; G.J. Prud'Homme et al., J. Exp. Med. 157, 730-742, 1983); Milzzellen-konditioniertes Medium (SCCM: E. Pure et al., J. Immunol. 127, 1953-1958, 1981)).

Inkubation der Kulturen erfolgte bei 37°C und 7,5 % $CO_2$ in feuchtigkeitsgesättigter Luft.

Le A 23 918

Nach 12-26 Tagen wurden die Mikrotiterplatten auf Wachstum von Klonen hin untersucht und die Näpfe mit Klonwachstum auf Anwesenheit von Antikörpern im Kulturüberstand mit Sandwich-Elisa's wie folgt geprüft. Anti-human IgG bzw. anti-human IgM Antikörper von der Ziege (Fa. Medac, Hamburg) wurden 1:100 mit 'phosphate-buffered saline' (PBS) verdünnt und in Microelisa-Platten (Immunolon II) durch Inkubation über Nacht bei 4°C gekoppelt (100 µl/Napf), und die Plastikoberfläche wurde mit 1 % Ovalbuminlösung abgesättigt. Dann wurden 50 µl Zellkulturüberstände oder humanes Kontroll-IgG bzw. -IgM (Medac) oder Kulturmedium zur Inkubation über Nacht bei 4°C zugegeben. Zum Nachweis spezifischer Bindung erfolgte die Beschichtung mit 50 µl anti-human IgG bzw. anti-human IgM Ziegenantikörpern, an die Peroxidase konjugiert war (1:200 in PBS-Tween 20 (2000:1) verdünnt; Medac) und 2 h Inkubation bei 37°C. Zwischen allen Schritten wurde jeweils 3-5 mal mit PBS/ Tween 20 gewaschen. Sodann wurden 50 µl einer $H_2O_2$/Chromogen-Lösung zugegeben (25 µl einer 0,1 %igen $H_2O_2$-Lösung in PBS pro ml ABTS-Lösung (2,2'-Azino-di-3-ethyl-benzthiazoline-Sulfonsäure; 1 mg/ml Citratpuffer pH 4)). Die Reaktion wurde mit 150 µl einer 0,5 %igen $NaN_3$-Lösung gestoppt und die Extinktion in einem Microelisa-Reader bei 405 nm gemessen.

Le A 23 918

| Zusatz | Näpfe | Näpfe mit Klonen | Klone | Zählung an Tag | IgM-pos. Überstände | IgG-pos. |
|--------|-------|------------------|-------|----------------|---------------------|----------|
| Ø | 192 | 116 | -- | 26 | 25 | 22 |
| TCM | 48 | 41 | -- | 12 | 15 | nd |
| TCM | 24 | -- | 19 | 10 | 0 | 0 |
| LNCM | 45 | -- | 78 | 21 | 10 | 26 |
| SCCM | 39 | -- | 53 | 13 | 4 | 7 |

Karyologische Untersuchungen zeigten, daß die Zahl der Chromosomen der Hybridome größer ist als die Zahl der Chromosomen von Lymphozyten oder Myelomzellen aber kleiner als die Summe der Chromosomen beider Fusionspartner.

Le A 23 918

Patentansprüche

1. Verfahren zur Förderung der Proliferation bei der Anzucht und/oder Expansion von Hybridomzellen, wobei man die mit bekannten Verfahren fusionierten Zellen mit einem Zellkulturmedium aufnimmt, in geeignete Zellkulturgefäße aussät und unter geeigneten Bedingungen bebrütet, dadurch gekennzeichnet, daß dem Zellkulturmedium ein mit geeigneten Zellen konditioniertes Zellkulturmedium zugesetzt wird.

2. Verfahren nach Anspruch 1, dadurch gekennzeichnet, daß die Hybridomzellen humane Hybridomzellen sind.

3. Verfahren nach Anspruch 1 oder 2, dadurch gekennzeichnet, daß für die Konditionierung mit Mitogen stimulierte Milzzellen von Mäusen verwendet werden.

4. Verfahren nach Anspruch 1 oder 2, dadurch gekennzeichnet, daß für die Konditionierung Lymphknotenzellen von homozygoten LPR-Mäusen verwendet werden.

5. Verfahren nach Anspruch 1 oder 2, dadurch gekennzeichnet, daß für die Konditionierung Thymozyten von jungen Mäusen verwendet werden.

6. Verfahren nach Anspruch 1, 2 oder 5, dadurch gekennzeichnet, daß für die Konditionierung Thymozyten von höchstens 2 Wochen alten Mäusen verwendet werden.

Le A 23 918

7. Verfahren nach den Ansprüchen 1 bis 6, dadurch gekennzeichnet, daß das Zellkulturmedium 10 bis 100 % des konditionierten Zellkulturmediums enthält.

8. Verfahren nach Anspruch 7 dadurch gekennzeichnet, daß das Zellkulturmedium ca. 50 % des konditionierten Zellkulturmediums enthält.

Le A 23 918